# EUROPEAN PATENT SPECIFICATION

(11) **EP 3 131 522 B1**
(45) Date of publication and mention of the grant of the patent: **25.10.2017**
(21) Application number: 15710534.7
(22) Date of filing: 19.03.2015
(51) Int. Cl.: A61K 8/25, A61K 8/34, A61K 8/73, A61K 8/898, A61K 8/06, A61Q 17/00, A61Q 13/00, A61Q 19/02, A61Q 19/08, A61Q 17/04, A61K 8/02

(54) **PERSONAL CARE COMPOSITION**
KÖRPERPFLEGEZUSAMMENSETZUNG
COMPOSITION POUR SOINS PERSONNELS

(30) Priority: 14.04.2014 WO PCT/CN2014/075304; 02.06.2014 EP 14170747
(43) Date of publication of application: 22.02.2017
(73) Proprietor: Unilever N.V., 3013 AL Rotterdam (NL); Unilever PLC, London, Greater London EC4Y 0DY (GB)
(72) Inventor: RAN, Li, Shanghai 201204 (CN); ZHANG, Qiqing, Shanghai 201615 (CN)
(74) Representative: Corsten, Michael Allan
(86) International application number: PCT/EP2015/055824
(87) International publication number: WO 2015/158491

(56) References cited:
- WO-A1-2010/046238
- WO-A1-2011/036048
- WO-A1-2011/137563
- WO-A1-2013/064365
- WO-A1-2013/064367
- WO-A1-2013/064599
- WO-A2-2013/064597
- "CTFA Monograph ID 115: Aluminium Starch Octenylsuccinate ED - Gottschalck T E; McEwen G N", 1 January 2006 (2006-01-01), INTERNATIONAL COSMETIC INGREDIENT DICTIONARY AND HANDBOOK; [INTRODUCTION, INCI NAME MONOGRAPHS A-I], THE COSMETIC, TOILETRY, AND FRAGRANCE ASSOCIATION, WASHINGTON, D.C, PAGE(S) 112, XP002725309, ISBN: 978-1-882621-36-1 the whole document

## Description

### Technical Field of the Invention

The invention concerns a water-in-oil personal care composition suitable for benefit agent delivery, particularly a personal care composition suitable for anti-microbial agent delivery. Moreover, the present invention also relates to the process for manufacturing such personal care compositions.

### Background of the Invention

Benefit agents such as anti-microbial agents, perfume, sunscreen agents, anti-aging agents, skin lightening agents are commonly incorporated in personal care compositions and are delivered to the skin of the human body to improve the skin condition. Due to the high cost of most benefit agents, it is desired to improve the delivery efficiencies of benefit agents to maximize the effectiveness of such benefit agents.
However, in personal care compositions, small molecules of benefit agents are likely to form micelle structure with surfactants that makes it difficult for them to be released. Thus it often takes long time to provide the desired benefits. For instant benefits such as instant bacteria killing, there is a need of providing a composition that offers a high benefit agent delivery efficiency. WO 2011/137563 A1 discloses emulsions comprising a functionalized polymer and a particle having an isoelectric point below the pH of the emulsion. Excellent sensory and active deliverability benefits are obtained.

The present inventors have now developed a water-in-oil personal care composition that can provide enhanced delivery efficiency of benefit agents especially anti-microbial agents. It has been found that this need can be met by using a combination of hydrophobically modified particles, amino functionalized silicone and an anti-microbial agent comprising monoterpenes. Additionally, it is further found that the process for manufacturing such compositions also affects the benefit agent delivery efficiency.

### Summary of the Invention

In a first aspect, the present invention is concerned with a water-in-oil personal care composition comprising:
a) hydrophobically modified particles selected from the group consisting of silica, metal oxide and mixtures thereof;
b) amino functionalized silicone; and
c) an anti-microbial agent comprising monoterpenes.

In a second aspect, the present invention is directed to a packaged personal care product comprising the personal care composition of the first aspect of this invention.

In a third aspect, the present invention is also directed to a process for manufacturing a water-in-oil personal care composition comprising hydrophobically modified particles selected from the group consisting of silica, metal oxide and mixtures thereof, amino functionalized silicone and an anti-microbial agent comprising monoterpenes, wherein the process comprises the steps of:
a) premixing the hydrophobically modified particles selected from the group consisting of silica, metal oxide and mixtures thereof, the amino functionalized silicone and the anti-microbial agent; and
b) mixing the premix with other ingredients to form the personal care composition.

In a fourth aspect, the present invention is concerned with a personal care composition obtainable and/or obtained by a process of the third aspect.

In a fifth aspect, the present invention is directed to a method of using the personal care composition of any embodiment of the first and the fourth aspects of this invention to provide enhanced benefit agent delivery.

All other aspects of the present invention will more readily become apparent upon considering the detailed description and examples which follow.

Except in the examples, or where otherwise explicitly indicated, all numbers in this description indicating amounts of material or conditions of reaction, physical properties of materials and/or use may optionally be understood as modified by the word "about". All amounts are by weight of the final personal care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Detailed Description

It has been found that a water-in-oil personal care composition comprising hydrophobically modified particles, amino functionalized silicone and an anti-microbial agent comprising monoterpenes can provide enhanced delivery efficiency of benefit agent especially anti-microbial agent. It is further found that the process for manufacturing a water-in-oil personal care composition comprising hydrophobically modified particles selected from the group consisting of silica, metal oxide and mixtures thereof, amino functionalized silicone and benefit agent also affects the benefit agent delivery efficiency.

Benefit agents as used herein means an active typically delivered to an external surface of the human body to enhance or improve a characteristic of the surface. For example, anti-microbial agents such as thymol, terpineol, eugenol and climbazole; sunscreen agent such as octyl methoxycinnamate, octocrylene, octyl salicylate, benzophenone-3 and avobenzone; anti-aging, wrinkle-reducing, skin lightening agents, anti-acne agents, and sebum reduction agents such as alpha-hydroxy acids and esters, beta-hydroxy acids and ester, polyhydroxy acids and esters, kojic acid and esters, ferulic acid and ferulate derivatives, vanillic acid and esters, dioic acids and esters, niacinamide, vitamin C and its derivatives, 12-hydroxystearic acid, retinol, retinal, retinyl esters, hydroquinone, t-butyl hydroquinone, mulberry extract, licorice extract, and resorcinol derivatives; perfume such as myrcene, dihydromyrcenol, citral, tagetone, cis-geranic acid, citronellic acid and cis-geranic acid nitrile.

The personal care composition of the present invention may comprise a single benefit agent or a mixture of two or more benefit agents. Typically, the benefit agent is present in an amount from 0.01 to 20%, more preferably from 0.03 to 15%, more preferably still from 0.05 to 10%, most preferably from 0.1 to 5% by total weight of the personal care composition and including all ranges subsumed therein.

Delivery efficiency as used herein means the ability to deliver benefit agents to an external surface of the human body through topical application.

The personal care composition of the invention is a water-in-oil emulsion. Generally the water content is from 10 to 80%, more preferably from 15 to 70%, most preferably from 20 to 60% based on the total weight of the personal care composition and including all ranges subsumed therein.

### Hydrophobically Modified Particles

The only limitation with respect to the type of hydrophobically modified particles that may be used in this invention is that the same is suitable for use in a personal care composition by consumers. The hydrophobically modified particle includes silica, metal oxide or mixtures thereof. Preferably the hydrophobically modified particle is hydrophobically modified silica.

In a preferred embodiment, the hydrophobically modified silica comprises at least one of the following groups:

-O-Si(CH₃)₃ (I)

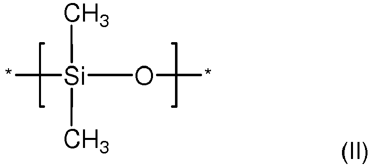

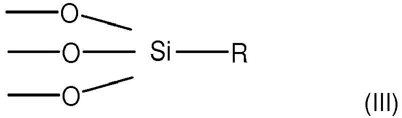

in which R is a C₄ to C₁₈ alkyl group.
or

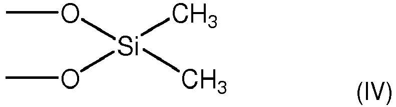

Such silicas are described in US 7 282 236 and made commercially available from suppliers like Evonik Degussa GmbH under the names Aerosil R805, R972, R 202, R812, R104, R816 and R711. Preferably, the silica particles comprise the group representing by formula III. More preferably, the silica particles comprise octylsilane (sold under the name Aerosil R805).

The hydrophobically modified particles according to the present invention can be of different sizes and shapes. Particle size, as used herein, refers to particle diameter unless otherwise stated. Diameter is meant to mean the largest measurable distance on a particle in the event a well-defined sphere is not generated. Particle size can be measured, for example, by dynamic light scattering (DLS). The size of hydrophobically modified particles is often from 1 nm to 100 nm, more preferably from 3 nm to 70 nm, most preferably from 5 nm to 20 nm, including all ranges subsumed therein.

Typically, the personal care composition of the present invention comprises from 0.05 to 20% by weight of the hydrophobically modified particles, more preferably from 0.1 to 15%, most preferably from 0.3 to 10%, based on the total weight of the personal care composition and including all ranges subsumed therein.

### Amino Functionalized Silicone

The personal care composition of this invention comprises an amino functionalized silicone. By "amino functionalized silicone" is meant a silicone containing at least one primary, secondary or tertiary amine group, or a quaternary ammonium group.

The primary, secondary, tertiary and/or quaternary amine groups may either form part of the main polymer chain or more preferably be carried by a side or pendant group carried by the polymeric backbone. Such polymers are described, for example, in US 4 185087.

In a preferred embodiment, the amino functionalized silicone has the general formula: 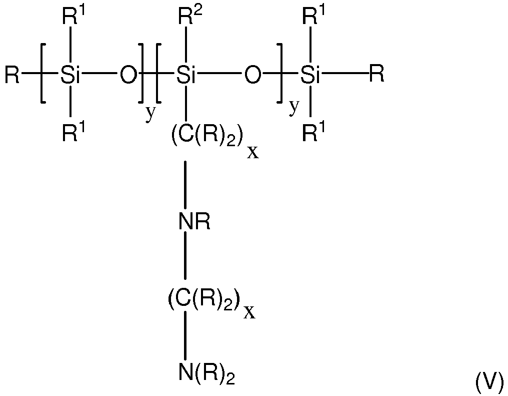 where:
each R is independently H, OH, OCH₃ or C₁₋₄ alkyl;
each R¹ is independently OR, or a C₁₋₄ alkyl;
each R² is independently OR, or a C₁₋₄ alkyl; and
each x is independently an integer from 1 to 4 and each y is greater than zero and independently an integer to yield a polymer having a molecular weight from 500 to 1 million, and preferably, from 750 to 25,000, and most preferably from 1,000 to 15,000.

Other amino functionalized silicone suitable to use includes silicone cationic polymers represented by the formula:

R²ₐZ₃₋ₐ-Si(OSiZ₂)ₙ-(OSiZ_{b}R²_{2-b})ₘ-O-SiZ₃₋ₐ-R²ₐ (VI)

where:
Z is hydrogen, phenyl, OH or a C₁₋C₁₀ alkyl group;
each a is independently an integer from 0 to 3;
b is an integer from 0 to 1; and
m and n are integers whereby the sum of n+m ranges from 1 to 3,500, and
preferably from 10 to 160;
each R² is independently a monovalent radical of formula -C_{q}H_{2q}L whre each q is independently a number from 2 to 10 and L is an amine or a quaternized amine represented by one of the following groups:

   -NR³-CH₂-CH₂-N(R³)₂

   -N(R³)₂

   -N^{⊕}(R³)₃A⁻

   -N(R³)-CH₂-CH₂-N⁺R³(H)₂A⁻,

   where:
   each R³ is independently hydrogen, phenyl, benzyl or a C₁ to C₁₂ alkyl; and
   each A⁻ is independently fluoride, chloride, bromide or iodide anion.

Still other amino functionalized silicone suitable for use includes those having the formula: 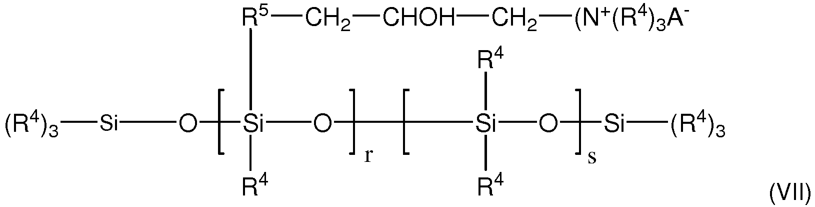 where:
each R⁴ is independently a C₁ to C₂₀ alkyl or C₂ to C₂₀ alkenyl;
R⁵ is a divalent C₁-C₁₈ group;
A⁻ is as previously defined;
r is an integer from 2 to 20; and
s is an integer from 15 to 75.

Preferably, the amino functionalized silicone has the INCI designation amodimethicone.

More preferably, the amodimethicone is represented by formula (V) and made commercially available, for example, as DC 8500 by Dow Corning. It is also within the scope of this invention for the amino functionalized silicone to comprise trimethylsilylamodimethicone represented by formula (VI).

Typically, the personal care composition of the present invention comprises amino functionalized silicone in an amount from 0.01 to 20%, more preferably from 0.03 to 15%, more preferably still from 0.05 to 10%, most preferably from 0.1 to 5% by total weight of the personal care composition and including all ranges subsumed therein.

Preferably the weight ratio of hydrophobically modified particle to amino functionalized silicone is from 10:1 to 1:30, more preferably from 5:1 to 1:10.

### Anti-microbial Agent

The personal care composition of the invention also comprises an anti-microbial agent comprising monoterpenes. Preferably, the anti-microbial agent is selected from thymol, terpineol, eugenol or mixtures thereof.

Thymol, terpineol and eugenol are all components of essential oils. It has been reported in WO2010/046238 that compositions comprising thymol and terpineol in selective range of concentrations provide relatively quick anti-microbial action. In WO2011/036048, reports that in the presence of eugenol, the fast anti-microbial action can even be achieved with a much lower concentration of thymol and terpineol.

The structure of thymol is given below: 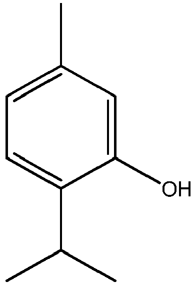 Thymol is a natural monoterpene phenol derivative of cymene. Typically, thymol is present in an amount from 0.01 to 5%, more preferably from 0.02 to 1%, most preferably from 0.05 to 0.5% by total weight of the personal care composition and including all ranges subsumed therein.

The structure of terpineol is given below: 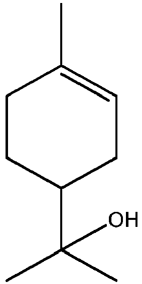 Terpineol is a natural monoterpene alcohol isolated from a variety of sources such as cajuput oil, pine oil and petitgrain oil. Terpineol has four isomers including alpha-terpineol, beta-terpineol, gamma-terpineol and terpinen-4-ol, wherein alpha-terpineol is preferred. Typically, terpineol is present in an amount from 0.01 to 10%, more preferably from 0.05 to 5%, most preferably from 0.1 to 1% by total weight of the personal care composition and including all ranges subsumed therein.
The structure of eugenol is given below: 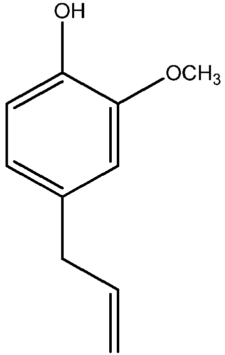 Eugenol is a phenylpropene, an allyl chain-substituted guaiacol extracted from a variety of sources such as clove oil, nutmeg, cinnamon, basil and bay leaf. Typically, eugenol is present in an amount from 0.005 to 5%, more preferably from 0.01 to 1%, most preferably from 0.02 to 0.5% by total weight of the personal care composition and including all ranges subsumed therein.

The personal care composition of the present invention may comprise a combination of thymol, terpineol and eugenol in any of the preferred concentrations as specified above for the three of them respectively.

### Other Ingredients

The personal care composition of the invention may be in any form including toners, lotions, creams, mousses, serum or gel that is suitable for topical application to the skin. The personal care composition can be either a leave-on or a rinse-off product, preferably a leave-on product, especially a skin care product including skin lotions and skin creams.

The personal care composition of the invention may comprise hydrophobic starch. Natural starches are usually modified physically or chemically to obtain modified starches. Physically modified starches include gelatinized starches, fully or partially hydrated starches and destructurized starches as well as crosslinked starches. Chemically modified varieties are those that have undergone, for example, acylation, alkylation, epoxidization, quaternization, carboxylation, phosphorylation, etherification (e.g. reaction with propylene or ethylene oxide), esterification (e.g. reaction with acetic anhydride). Generally, hydrophobic starch is starch which has been modified to impart hydrophobic groups that render the starch hydrophobic in nature. Preferably hydrophobic starches are starch esters containing hydrophobic groups and complex ethers of starch such as aluminium starch octenyl succinate.

The hydrophobic starch is generally present in personal care composition of this invention in an amount from 0.01 to 15%, more preferably from 0.03 to 10%, most preferably from 0.05 to 5%, based on the total weight of the personal care composition and including all ranges subsumed therein.

The personal care composition may comprise non-amino functionalized silicone oils as the oil continuous phase. Suitable silicones include polydiorganosiloxanes, in particular polydimethylsiloxanes which have the CTFA designation dimethicone. Also suitable for use in compositions of this invention are polydimethyl siloxanes having hydroxyl end groups, which have the CTFA designation dimethiconol. Also suitable for use in compositions of this invention are silicone gums having a slight degree of crosslinking, as are described for example in WO 96/31188. Especially preferred is crosslinked silicone elastomer blend. Suitable silicone elastomer blends which are commercially available include DC 9041 (dimethicone and dimethicone crosspolymer from Dow Corning), DC 9045 and DC 9040 (cyclopentasiloxane and dimethicone crosspolymer from Dow Corning) DC 9546 (cyclopentasiloxane and dimethicone crosspolymer and dimethicone/vinyldimethicone crosspolymer and dimethiconol, from Dow Corning), DC 9506 (dimethicone/vinyldimethicone crosspolymer from Dow Corning); silicone elastomer suspensions like DC 9509 (dimethicone/vinyldimethicone crosspolymer and C12-14 Pareth-12 from Dow Corning).

The amount of the non-amino functionalized silicone oil is preferably in the range from 0.05 to 60%, more preferably from 1 to 50% and most preferably from 5 to 40% by total weight of the personal care composition and including all ranges subsumed therein.

The personal care composition may additionally comprise emulsifiers, preferably oil soluble emulsifiers. Suitable emulsifiers are selected from fatty acids, fatty alcohols, polyglycerol fatty acid esters, sucrose fatty acid esters, glycerol fatty acid esters, propylene glycol fatty acid esters, sorbitan fatty acid esters, lecithin or mixtures thereof. For example, caprylic/capric triglyceride, cetyl alcohol, sucrose distearate, polyglyceryl oleate, glyceryl monooleate, sorbitan stearate, sorbitan monooleate. Typically, the emulsifier is present in an amount ranging from 0.1 to 15%, more preferably from 0.2 to 10%, most preferably from 0.5 to 5% by total weight of the personal care composition and including all ranges subsumed therein.

The personal care composition may further comprise other ingredients which are common in the art to enhance physical properties and performances. Suitable ingredients include but are not limited to surfactants, thickeners, humectants, opacifiers, binders, colorants and pigments, pH adjusting agents, viscosity modifiers, preservatives, biological additives, buffering agents, conditioners, natural extracts and essential oils.

In addition to water, organic solvent may also be included to act as co-solvent. Preferably the organic solvent is a polyhydric alcohol like glycerin (i.e. glycerol), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Preferably the polyhydric alcohol is propylene glycol.

Typically, the personal care composition of the present invention comprises co-solvent in an amount from 0.05 to 20%, more preferably from 0.1 to 15%, most preferably from 0.5 to 10%, based on the total weight of the personal care composition and including all ranges subsumed therein.

A wide variety of packaging can be employed to store and deliver the personal care compositions. Packaging is often dependent upon the type of personal care end-use. For instance, leave-on skin lotions and creams, shampoos, hair conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered as a stick composition formulation in a container with propel-repel mechanism where the stick moves on a platform towards a dispensing orifice. Metallic cans pressurized by a propellant and having a spray nozzle serve as packaging for antiperspirants, shave creams and other sprayable personal care products. Toilet bars may have packaging constituted by a cellulosic or plastic wrapper or within a cardboard box or even encompassed by a shrink wrap plastic film.

### Method for Manufacturing Personal Care Compositions

This invention is also directed to a method for manufacturing a water-in-oil personal care composition. Preferably, the method comprises the steps of:
a) premixing hydrophobically modified particles selected from the group consisting of silica, metal oxide and mixtures thereof, amino functionalized silicone and benefit agent; and
b) mixing the premix with other ingredients to form the personal care composition. The benefit agent is an anti-microbial agent comprising monoterpenes.

Additionally or alternatively, the premix of step (a) comprises from 1 to 10% by weight of hydrophobically modified particles selected from the group consisting of silica, metal oxide and mixtures thereof.
Additionally or alternatively, the premix of step (a) comprises from 0.5 to 10% by weight of amino functionalized silicone.
Additionally or alternatively, the premix of step (a) comprises from 0.5 to 10% by weight of benefit agent.

In an especially preferred embodiment, the premix of step (a) comprises hydrophobic starch. Preferably the hydrophobic starch is present in an amount from 0.2 to 10% by total weight of the premix of step (a).

The invention is also concerned with the personal care composition obtainable and/or obtained by the method for manufacturing the personal care composition.

The invention is further concerned with a method of using the personal care composition to provide enhanced benefit agent delivery.

The following examples are provided to facilitate an understanding of the present invention. The examples are not provided to limit the scope of the claims.

### Examples

### Example 1

A number of samples were made to demonstrate aspects of the present invention. The detailed formulations are outlined in Table 1. All ingredients are expressed by weight percent of the total formulation and as level of active ingredient.

**Table 1**

| Phase | Trade Name (INCI Name) | Samples | | | | | | | | |
|---|---|---|---|---|---|---|---|---|---|---|
| | | 1 | 2 | 3 | 4 | 5 | 6 | 7 | 8 | 9 |
| A | Parsol MCX (Ethylhexyl methoxycinnamate & BHT) | 2.00 | 2.00 | 2.00 | 2.00 | 2.00 | 2.00 | 2.00 | 2.00 | 2.00 |
| | DC 9045 (Cyclopentasiloxane and dimethicone crosspolymer) | 26.50 | 26.50 | 26.50 | 26.50 | 26.50 | 26.50 | 26.50 | 26.50 | 26.50 |
| B | CRODAMOL GTCC-LQ-(SG) (Caprylic/capric triglyceride) | 2.50 | 2.50 | 2.50 | 2.50 | 2.50 | 2.50 | 2.50 | 2.50 | 2.50 |
| | Silsoft 034 (Caprylyl methicone) | 0.50 | 0.50 | 0.50 | 0.50 | 0.50 | 0.50 | 0.50 | 0.50 | 0.50 |
| | Parsol MCX (Ethylhexyl methoxycinnamate & BHT) | 2.00 | 2.00 | 2.00 | 2.00 | 2.00 | 2.00 | 2.00 | 2.00 | 2.00 |
| | CHOLESTEROL NF (Cholesterol) | 0.20 | 0.20 | 0.20 | 0.20 | 0.20 | 0.20 | 0.20 | 0.20 | 0.20 |
| | SA-1840 (Stearic, palmitic, myristic, lauric & oleic acid) | 0.25 | 0.25 | 0.25 | 0.25 | 0.25 | 0.25 | 0.25 | 0.25 | 0.25 |
| | Cetyl Alcohol (Cetyl alcohol) | 0.01 | 0.01 | 0.01 | 0.01 | 0.01 | 0.01 | 0.01 | 0.01 | 0.01 |
| | Crodesta F-10 (Sucrose distearate) | 0.132 | 0.132 | 0.132 | 0.132 | 0.132 | 0.132 | 0.132 | 0.132 | 0.132 |
| C | KF-6017 (PEG-10 dimethicone) | 1.188 | 1.188 | 1.188 | 1.188 | 1.188 | 1.188 | 1.188 | 1.188 | 1.188 |
| | DC 245 (Cyclopentasiloxane) | 0.50 | 0.50 | 0.50 | 0.50 | 0.50 | 0.50 | 0.50 | 0.50 | 0.50 |
| | Bentone 38 VCG (Disteardimonium Hectorite) | 0.27 | 0.27 | 0.27 | 0.27 | 0.27 | 0.27 | 0.27 | 0.27 | 0.27 |
| | JTTO-MS7 (Titanium dioxide, alumina & methicone) | 2.00 | 2.00 | 2.00 | 2.00 | 2.00 | 2.00 | 2.00 | 2.00 | 2.00 |
| | Timiron Ultra Luster MP 111 (Mica & titanium dioxide) | 0.50 | 0.50 | 0.50 | 0.50 | 0.50 | 0.50 | 0.50 | 0.50 | 0.50 |
| | SA-TR-10 (Titanium dioxide & dimethicone) | 0.60 | 0.60 | 0.60 | 0.60 | 0.60 | 0.60 | 0.60 | 0.60 | 0.60 |
| | Parsol MCX (Ethylhexyl methoxycinnamate & BHT) | 2.00 | 2.00 | 2.00 | 2.00 | 2.00 | 2.00 | 2.00 | 2.00 | 2.00 |
| D | Glycerin 99.5% USP (Glycerin) | 7.25 | 7.25 | 7.25 | 7.25 | 7.25 | 7.25 | 7.25 | 7.25 | 7.25 |
| | Disodium EDTA (Disodium EDTA) | 0.05 | 0.05 | 0.05 | 0.05 | 0.05 | 0.05 | 0.05 | 0.05 | 0.05 |
| | Potassium chloride (Potassium chloride) | 1.00 | 1.00 | 1.00 | 1.00 | 1.00 | 1.00 | 1.00 | 1.00 | 1.00 |
| | Magnesium sulfate heptahydrate (Magnesium sulfate) | 0.50 | 0.50 | 0.50 | 0.50 | 0.50 | 0.50 | 0.50 | 0.50 | 0.50 |
| | Niacinamide PC (Niacinamide) | 3.00 | 3.00 | 3.00 | 3.00 | 3.00 | 3.00 | 3.00 | 3.00 | 3.00 |
| | Incromectant AMEA 100 (Acetamide MEA) | 0.05 | 0.05 | 0.05 | 0.05 | 0.05 | 0.05 | 0.05 | 0.05 | 0.05 |
| | Purasal NH/COS (Ammonium lactate) | 0.071 | 0.071 | 0.071 | 0.071 | 0.071 | 0.071 | 0.071 | 0.071 | 0.071 |
| | D&C RED No 33 (Cl 17200) | 5x10⁻⁴ | 5x10⁻⁴ | 5x10⁻⁴ | 5x10⁻⁴ | 5x10⁻⁴ | 5x10⁻⁴ | 5x10⁻⁴ | 5x10⁻⁴ | 5x10⁻⁴ |
| | Glydant Plus Liquid (DMDM hydantoin, IPBC, butylene glycol & water) | 0.20 | 0.20 | 0.20 | 0.20 | 0.20 | 0.20 | 0.20 | 0.20 | 0.20 |
| E | Thymol (2-isopropyl-5-methylphenol) | 0.2 | 0.2 | 0.2 | 0.2 | 0.2 | 0.2 | 0.2 | -- | 0.2 |
| | Terpineol [2-(4-methyl-1-cyclohex-3-enyl) propan-2-ol] | 0.5 | 0.5 | 0.5 | 0.5 | 0.5 | 0.5 | 0.5 | -- | 0.5 |
| | Eugenol (4-Allyl-2-methoxyphenol) | 0.05 | 0.05 | 0.05 | 0.05 | 0.05 | 0.05 | 0.05 | -- | 0.05 |
| | PG (Propylene glycol) | -- | 2.00 | 2.00 | 2.00 | 2.00 | -- | 2.00 | 2.00 | -- |
| | Aerosil R805 (Hydrophobic silica) | -- | -- | 0.90 | -- | 0.90 | 0.90 | 0.90 | 0.90 | 0.90 |
| | Amino Si 8500 | -- | -- | 0.45 | 0.45 | -- | 0.45 | 0.45 | 0.45 | 0.45 |
| | (Bis(C13-C15 alkoxy) PG amodimethicone) | | | | | | | | | |
| F | Water and minors | To 100 | To 100 | To 100 | To 100 | To 100 | To 100 | To 100 | To 100 | To 100 |

### Example 2

This example demonstrates the bio-efficacy of compositions for bacterial killing.

### Formulation Process

Phase A was first added into the main pot and mixed at 50-55°C. Phase B was heated to 80-85°C and uniformly mixed, then cooled down to 70°C, followed by adding ingredients of Phase C. The mixture of Phase B and C were then added into the main pot to mix with Phase A at 50-55°C. Phase D was premixed at 50-55°C till all the ingredients dissolved and the mixture was slowly added into the main pot and mixed. The final mixture of Phase A, B, C and D was homogenized for 15-20 minutes at 50-55°C before cooled down for use. All the ingredients of Phase E were premixed to form a premix first before the resulting premix was dispersed into the mixture of Phase A-D. Phase F was added and mixed into the mixture of Phase A-E at last. The process was carried out under ambient (25°C) temperature.

### Assessment for Bio-Efficacy

Vitro-Skin™ was sandwiched in a plastic ring support with its rough topography facing up. Microorganisms for the inoculation of artificial skin were initially grown in Trypticase Soy Broth, and adjusted to a final concentration of 2-6×10⁶ cells/mL prior to the inoculation of the Vitro-Skin™. 0.2 mL of microorganisms was gently pipetted onto the rough surface of the skin to form a film with a depth of 1-2 mm. Testing sample was added to the plastic ring and stirred with a Teflon stirring rod for 1 minute. Following the treatment, the sampling was conducted. Each piece of Vitro-Skin™ was carefully folded in half using sterile forceps, and placed a vial containing 10 mL of Dey/Engley Neutralizing Broth (Sigma). The vial was vortexed for 1 minute and then treated ultrasonically for 1 minute. 20 µL of 10°-10⁻³ dilutions were placed onto Trypticase Soy Agar plates and anaerobic cultivation at 37°C for 3 days. The number of colonies on each plate was then counted, and final numbers were determined by multiplying by the appropriate dilution.

In-vitro anti-propionibacterium acnes results are shown in Table 2.

**Table 2**

| | Samples | | | | | | | | |
|---|---|---|---|---|---|---|---|---|---|
| | 1 | 2 | 3 | 4 | 5 | 6 | 7 | 8 | 9 |
| Mean Log Reduction | -0.09 | -0.22 | -0.53 | -0.33 | -0.26 | -0.36 | -0.39 | -0.11 | -0.27 |
| Standard Deviation | 0.03 | 0.04 | 0.09 | 0.04 | 0.02 | 0.03 | 0.05 | 0.06 | 0.03 |

Sample 3, which is an embodiment of the present invention, demonstrates significantly better anti-bacteria efficacy than Sample 2 which does not comprise hydrophobically modified particles and amino functionalized silicone.

Sample 4 and Sample 5 comprise either hydrophobically modified particles or amino functionalized silicone, both of which demonstrate lower anti-bacteria efficacy compared to Sample 3.

Moreover, it is surprisingly found that the formulation process can have effect on the anti-bacteria efficacy of the composition.

Samples 7 and 9 were prepared by adding ingredients of Phase E into the mixture of Phase A-D one by one. All other samples were prepared by premixing all the ingredients of Phase E first to form a premix before adding the resulting premix into the mixture of Phase A-D to obtain the final composition. Sample 3 showed significantly improved anti-bacteria efficacy compared to Sample 7, which indicates that premixing all the ingredients of Phase E to form a premix before adding them into the mixture of Phase A-D is a preferred formulation process.

### Example 3

This example demonstrates the stability of the composition.

### Rheology Measurement Method

Samples were stocked in a water bath at 25°C and their viscosities were measured after 2 and 24 hours. They were measured by DV-I+PRO Digital Viscometer (Brookfield Ltd) at a speed of 5 rpm after 30 seconds.

**Table 3**

| Sample | Viscosity after 2 hours storage at 25°C (mPa•s) | Viscosity after 24 hours storage at 25°C (mPa•s) |
|---|---|---|
| 3 (without Phase E) | 66560 | 64100 |
| 3 | 68000 | 72320 |

Table 3 shows that the composition according to the present invention is stable.

## Claims

1. A water-in-oil personal care composition comprising:
a) hydrophobically modified particles selected from the group consisting of silica, metal oxide and mixtures thereof;
b) amino functionalized silicone; and
c) an anti-microbial agent comprising monoterpenes.

2. The personal care composition according to claim 1, wherein the hydrophobically modified particle is hydrophobically modified silica.

3. The personal care composition according to claim 1 or claim 2, wherein the hydrophobically modified silica comprises: 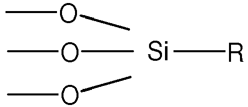 wherein R is a C₄ to C₁₈ alkyl group, preferably R is C₈H₁₇ alkyl group.

4. The personal care composition according to any of the preceding claims, wherein the amino functionalized silicone comprises amodimethicone.

5. The personal care composition according to any of the preceding claims, wherein the composition comprises amino functionalized silicone in an amount from 0.01 to 20%, preferably from 0.05 to 10% by total weight of the composition.

6. The personal care composition according to any of the preceding claims, wherein the weight ratio of hydrophobically modified particle to amino functionalized silicone is from 10:1 to 1:30, preferably from 5:1 to 1:10.

7. The personal care composition according to any of the preceding claims, wherein the anti-microbial agent is selected from thymol, terpineol, eugenol or mixtures thereof.

8. The personal care composition according to claim 7, wherein the composition comprises thymol in an amount from 0.01 to 5%, preferably from 0.02 to 1% by total weight of the composition.

9. The personal care composition according to claim 7 or claim 8, wherein the composition comprises terpineol in an amount from 0.01 to 10%, preferably from 0.05 to 5% by total weight of the composition.

10. The personal care composition according to any one of claims 7 to 9, wherein the composition comprises eugenol in an amount from 0.005 to 5%, preferably from 0.01 to 1% by total weight of the composition.

11. The personal care composition according to any of the preceding claims, wherein the composition further comprises hydrophobic starch, preferably in an amount from 0.03 to 10% by total weight of the composition.

12. The personal care composition according to any of the preceding claims, wherein the personal care composition further comprises a co-solvent, preferably propylene glycol.

13. A process for manufacturing a water-in-oil personal care composition which comprises hydrophobically modified particles selected from the group consisting of silica, metal oxide and mixtures thereof, amino functionalized silicone and an anti-microbial agent comprising monoterpenes , wherein the process comprises the steps of:
a) premixing the hydrophobically modified particles selected from the group consisting of silica, metal oxide and mixtures thereof, the amino functionalized silicone and the anti-microbial agent; and
b) mixing the premix with other ingredients to form the personal care composition.

14. The process according to claim 13, wherein the premix of step (a) comprises from 1 to 10% by weight of hydrophobically modified particles selected from the group consisting of silica, metal oxide and mixtures thereof.

15. The process according to claim 13 or claim 14, wherein the premix of step (a) further comprises hydrophobic starch.

## Patentansprüche

1. Wasser-in-ÖI-Körperpflegezusammensetzung, umfassend:
a) hydrophob modifizierte Partikel, die aus der Gruppe ausgewählt sind, die aus Siliziumdioxid, Metalloxid und Mischungen davon besteht,
b) aminofunktionalisiertes Silikon und
c) ein antimikrobielles Mittel, das Monoterpene umfasst.

2. Körperpflegezusammensetzung nach Anspruch 1, wobei das hydrophob modifizierte Partikel hydrophob modifiziertes Siliziumdioxid ist.

3. Körperpflegezusammensetzung nach Anspruch 1 oder Anspruch 2, wobei das hydrophob modifizierte Siliziumdioxid 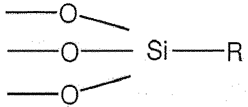 umfasst,
wobei R eine C₄- bis C₁₈-Alkylgruppe, vorzugsweise R eine C₈H₁₇-Alkylgruppe darstellt.

4. Körperpflegezusammensetzung nach irgendeinem der vorhergehenden Ansprüche, wobei das aminofunktionalisierte Silikon Amodimethicon umfasst.

5. Körperpflegezusammensetzung nach irgendeinem der vorhergehenden Ansprüche, wobei die Zusammensetzung aminofunktionalisiertes Silikon in einer Menge von 0,01 bis 20 Gewichts-%, vorzugsweise von 0,05 bis 10 Gewichts-% der gesamten Zusammensetzung umfasst.

6. Körperpflegezusammensetzung nach irgendeinem der vorhergehenden Ansprüche, wobei das Gewichtsverhältnis des hydrophob modifizierten Partikels zum aminofunktionalisierten Silikon von 10:1 bis 1:30, vorzugsweise von 5:1 bis 1:10, beträgt.

7. Körperpflegezusammensetzung nach irgendeinem der vorhergehenden Ansprüche, wobei das antimikrobielle Mittel unter Thymol, Terpineol, Eugenol oder Mischungen davon ausgewählt ist.

8. Körperpflegezusammensetzung nach Anspruch 7, wobei die Zusammensetzung Thymol in einer Menge von 0,01 bis 5 Gewichts-%, vorzugsweise von 0,02 bis 1 Gewichts-% der gesamten Zusammensetzung umfasst.

9. Körperpflegezusammensetzung nach Anspruch 7 oder Anspruch 8, wobei die Zusammensetzung Terpineol in einer Menge von 0,01 bis 10 Gewichts-%, vorzugsweise von 0,05 bis 5 Gewichts-% der gesamten Zusammensetzung umfasst.

10. Körperpflegezusammensetzung nach irgendeinem der Ansprüche 7 bis 9, wobei die Zusammensetzung Eugenol in einer Menge von 0,005 bis 5 Gewichts-%, vorzugsweise von 0,01 bis 1 Gewichts-% der gesamten Zusammensetzung umfasst.

11. Köperpflegezusammensetzung nach irgendeinem der vorhergehenden Ansprüche, wobei die Zusammensetzung ferner hydrophobe Stärke, vorzugsweise in einer Menge von 0,03 bis 10 Gewichts-% der gesamten Zusammensetzung, umfasst.

12. Körperpflegezusammensetzung nach irgendeinem der vorhergehenden Ansprüche, wobei die Körperpflegezusammensetzung ferner ein Cosolvens, vorzugsweise Propylenglykol, umfasst.

13. Verfahren zur Herstellung einer Wasser-in-ÖI-Körperpflegezusammensetzung, die hydrophob modifizierte Partikel umfasst, die aus der Gruppe ausgewählt sind, die aus Siliziumdioxid, Metalloxid und Mischungen davon, aminofunktionalisiertem Silikon und einem Monoterpene umfassenden antimikrobiellen Mittel besteht, wobei das Verfahren die Schritte umfasst:
a) Vormischen der hydrophob modifizierten Partikel, die aus der Gruppe ausgewählt sind, die aus Siliziumdioxid, Metalloxid und Mischungen davon, dem aminofunktionalisierten Silikon und dem antimikrobiellen Mittel besteht, und
b) Mischen der Vormischung mit anderen Bestandteilen, um die Körperpflegezusammensetzung zu bilden.

14. Verfahren nach Anspruch 13, wobei die Vormischung des Schritts (a) von 1 bis 10 Gewichts-% hydrophob modifizierte Partikel umfasst, die aus der Gruppe ausgewählt sind, die aus Siliziumdioxid, Metalloxid und Mischungen davon besteht.

15. Verfahren nach Anspruch 13 oder Anspruch 14, wobei die Vormischung des Schritts (a) ferner hydrophobe Stärke umfasst.

## Revendications

1. Composition d'hygiène corporelle de type eau dans l'huile comprenant :
a) des particules modifiées par substitution hydrophobe choisies dans le groupe constitué par la silice, un oxyde métallique et leurs mélanges ;
b) une silicone à fonction amino ; et
c) un agent antimicrobien comprenant des monoterpènes.

2. Composition d'hygiène corporelle selon la revendication 1, dans laquelle la particule modifiée par substitution hydrophobe est une silice modifiée par substitution hydrophobe.

3. Composition d'hygiène corporelle selon la revendication 1 ou la revendication 2, dans laquelle la silice modifiée par substitution hydrophobe comprend : 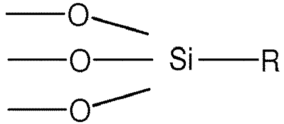 où R est un groupe alkyle C₄ à C₁₈, de préférence R est un groupe alkyle C₈H₁₇.

4. Composition d'hygiène corporelle selon l'une quelconque des revendications précédentes, dans laquelle la silicone à fonction amino comprend de l'amodiméthicone.

5. Composition d'hygiène corporelle selon l'une quelconque des revendications précédentes, dans laquelle la composition comprend la silicone à fonction amino en une quantité de 0,01 à 20 %, de préférence de 0,05 à 10 % en poids total de la composition.

6. Composition d'hygiène corporelle selon l'une quelconque des revendications précédentes, dans laquelle le rapport en poids de la particule modifiée par substitution hydrophobe à la silicone à fonction amino est de 10:1 à 1:30, de préférence de 5:1 à 1:10.

7. Composition d'hygiène corporelle selon l'une quelconque des revendications précédentes, dans laquelle l'agent antimicrobien est choisi parmi le thymol, le terpinéol, l'eugénol ou leurs mélanges.

8. Composition d'hygiène corporelle selon la revendication 7, dans laquelle la composition comprend du thymol en une quantité de 0,01 à 5 %, de préférence de 0,02 à 1 % en poids total de la composition.

9. Composition d'hygiène corporelle selon la revendication 7 ou la revendication 8, dans laquelle la composition comprend du terpinéol en une quantité de 0,01 à 10 %, de préférence de 0,05 à 5 % en poids total de la composition.

10. Composition d'hygiène corporelle selon l'une quelconque des revendications 7 à 9, dans laquelle la composition comprend de l'eugénol en une quantité de 0,005 à 5 %, de préférence de 0,01 à 1 % en poids total de la composition.

11. Composition d'hygiène corporelle selon l'une quelconque des revendications précédentes, dans laquelle la composition comprend en outre de l'amidon hydrophobe, de préférence en une quantité de 0,03 à 10 % en poids total de la composition.

12. Composition d'hygiène corporelle selon l'une quelconque des revendications précédentes, dans laquelle la composition d'hygiène corporelle comprend en outre un co-solvant, de préférence du propylène glycol.

13. Procédé de production d'une composition d'hygiène corporelle de type eau dans l'huile qui comprend des particules modifiées par substitution hydrophobe choisies dans le groupe constitué par la silice, un oxyde métallique et leurs mélanges, une silicone à fonction amino et un agent antimicrobien comprenant des monoterpènes, dans lequel le procédé comprend les étapes suivantes :
a) le prémélange des particules modifiées par substitution hydrophobe choisies dans le groupe constitué par la silice, un oxyde métallique et leurs mélanges, de la silicone à fonction amino et de l'agent antimicrobien ; et
b) le mélange du prémélange avec d'autres ingrédients pour former la composition d'hygiène corporelle.

14. Procédé selon la revendication 13, dans lequel le prémélange de l'étape (a) comprend de 1 à 10 % en poids de particules modifiées par substitution hydrophobe choisies dans le groupe constitué par la silice, un oxyde métallique et leurs mélanges.

15. Procédé selon la revendication 13 ou la revendication 14, dans lequel le prémélange de l'étape (a) comprend en outre un amidon hydrophobe.
